# EUROPEAN PATENT APPLICATION

(11) **EP 4 523 617 A2**
(43) Date of publication of application: **19.03.2025**
(21) Application number: 25154882.2
(22) Date of filing: 02.02.2018
(51) Int. Cl.: A61B 5/0537

(54) **BISYMMETRIC COMPARISON OF SUB-EPIDERMAL MOISTURE VALUES**

(30) Priority: 03.02.2017 US 201762454455 P; 19.06.2017 US 201762521871 P
(62) Divisional of application: 22172017.0
(71) Applicant: Bruin Biometrics, LLC, Los Angeles, CA 90024 (US)
(72) Inventor: BURNS, Martin F., Los Angeles, California, 90024 (US); BARRINGTON, Sara, Los Angeles, California 90024 (US); ROSS, Graham O., Los Angeles, California 90024 (US)
(74) Representative: Uexküll & Stolberg

(57) **Abstract**

The present disclosure provides apparatuses and methods for measuring sub-epidermal moisture at bisymmetric locations on patients to identify damaged tissue for clinical intervention.

## Description

### FIELD

The present disclosure provides apparatuses and computer readable media for measuring sub-epidermal moisture in patients to identify damaged tissue for clinical intervention. The present disclosure also provides methods for determining damaged tissue.

### BACKGROUND

The skin is the largest organ in the human body. It is readily exposed to different kinds of damages and injuries. When the skin and its surrounding tissues are unable to redistribute external pressure and mechanical forces, ulcers may be formed. Prolonged continuous exposure to even modest pressure, such as the pressure created by the body weight of a supine patient on their posterior skin surfaces, may lead to a pressure ulcer. In the presence of other damage, such as the neuropathy and peripheral tissue weakening that can be induced by diabetes, even periodic exposure to moderate levels of pressure and stress may lead to an ulcer, for example a foot ulcer.

Pressure ulcers are developed by approximately 2.5 million people a year in the United States and an equivalent number in the European Union. In long-term and critical-care settings, up to 25% of elderly and immobile patients develop pressure ulcers. Approximately 60,000 U.S. patients die per year due to infection and other complications from pressure ulcers.

Detecting tissue damage before the skin breaks and intervening with the appropriate therapy to avoid further deterioration of the underlying tissue is desirable not only for the patient but society. The average cost of treating pressure-induced damage at the earliest visible sign (a Stage 1 ulcer) is only $2,000 but this rises to $129,000 when the ulcer is deep enough to expose muscle or bone (a Stage 4 ulcer.) The current standard to detect pressure ulcers is by visual inspection, which is subjective, unreliable, untimely, and lacks specificity.

### SUMMARY

In an aspect, the present disclosure provides for, and includes, an apparatus for identifying damaged tissue, the apparatus comprising: a first and a second sensors, where the sensors each comprises a first electrode and a second electrode, and where each of the sensors is configured to be placed against a patient's skin; a circuit electronically coupled to the first and second electrodes and configured to measure an electrical property between the first and second electrodes of each of the sensors and provide information regarding the electrical property; a processor electronically coupled to the circuit and configured to receive the information from the circuit and convert the information into a sub-epidermal moisture (SEM) value; and a non-transitory computer-readable medium electronically coupled to the processor and comprising instructions stored thereon that, when executed on the processor, perform the step of: determining a difference between a first SEM value corresponding to the electrical property as measured by the first sensor at a first location on the patient's skin and a second SEM value corresponding to the electrical property as measured by the second sensor at a second location on the patient's skin, where the second location is bisymmetric relative to the first location.

In an aspect, an apparatus for identifying damaged tissue is provided by the present disclosure, the apparatus comprising: a substrate configured to be placed against a surface of a patient's skin; a plurality of sensors that are disposed on the substrate at a respective plurality of positions, where each sensor comprises a pair of electrodes; a circuit electronically coupled to the pair of electrodes of each of the plurality of sensors and configured to measure an electrical property between the pairs of electrodes of a portion of the plurality of sensors and provide information regarding the measured electrical properties; a processor electronically coupled to the circuit and configured to receive the information regarding the electrical properties from the circuit and convert the plurality of electrical properties into a respective plurality of sub-epidermal moisture (SEM) values; and a non-transitory computer-readable medium electronically coupled to the processor and comprising instructions stored thereon that, when executed on the processor, perform the steps of: identifying from the plurality of SEM values a first sensor and a second sensor that are located at first and second positions that are bisymmetric with respect to the patient's skin, and comparing a first SEM value that is associated with the first sensor with a second SEM value that is associated with the second sensor.

In one aspect, an apparatus for identifying damaged tissue is provided by the present disclosure, the apparatus comprising: an apparatus body; two sensors comprising a first sensor and a second sensor, where the two sensors are disposed on the apparatus body to allow simultaneous positioning of the first sensor on a first location on a patient's skin and the second sensor on a second location bisymmetric relative to the first location; a circuit electronically coupled to each of the two sensors and configured to measure an electrical property from each of the two sensors; a processor electronically coupled to the circuit and is configured to receive a first electrical property measurement from a first location and a second electrical property measurement from a second location, and to convert the first electrical property measurement to a first SEM value and the second electrical property measurement into a second SEM value; a non-transitory computer-readable medium electronically coupled to the processor and contains instructions that, when executed on the processor, perform the step of determining a difference between the first SEM value and the second SEM value.

In an aspect, a method for identifying damaged tissue is provided by the present disclosure, the method comprising: obtaining a first sub-epidermal moisture (SEM) value from a first location on a patient's skin; obtaining a second SEM value from a second location that is bisymmetric relative to the first location; determining a difference between a first SEM value and a second SEM value.

### BRIEF DESCRIPTION OF THE DRAWINGS

Aspects of the disclosure are herein described, by way of example only, with reference to the accompanying drawings. With specific reference now to the drawings in detail, it is stressed that the particulars shown are by way of example and are for purposes of illustrative discussion of aspects of the disclosure. In this regard, the description and the drawings, considered alone and together, make apparent to those skilled in the art how aspects of the disclosure may be practiced.
Figure 1A is an illustration of a plan view of a toroidal sensor.
Figure 1B illustrates a cross-section of the toroidal sensor of Figure 1A.
Figure 1C illustrates an idealized field map created by the toroidal sensor of Figure 1A when activated.
Figure 2A provides an example of a pair of bisymmetric locations on a sacral region according to the present disclosure.
Figure 2B provides an example of a pair of bisymmetric locations on the bottom side of both feet according to the present disclosure.
Figure 2C provides an example of a pair of bisymmetric locations on the lateral sides and soles of both feet according to the present disclosure.
Figure 3 is an illustration of an apparatus comprising one coaxial sensor.
Figure 4A is a first exemplary apparatus comprising two sensors according to the present disclosure.
Figure 4B is a second exemplary apparatus comprising two sensors and is configured to determine SEM values at bisymmetric locations according to the present disclosure.
Figure 5 is an exemplary apparatus comprising a plurality of sensors according to the present disclosure.
Figure 6 is a first exemplary array of electrodes.
Figure 7 is an exemplary array of electrodes according to the present disclosure.
Figure 8A illustrates a first example of how the array of electrodes disclosed in Figure 7 is configured to form a sensor according to the present disclosure.
Figure 8B illustrates a second example of how the array of electrodes disclosed in Figure 7 is configured to form a sensor according to the present disclosure.
Figure 9A illustrates an example of a first sensor formed in an array of electrodes according to the present disclosure.
Figure 9B illustrates an example of how a second sensor is formed to overlap with the first sensor of Figure 9A according to the present disclosure.
Figure 10 shows an example of how sensors as shown in Figure 8A are formed from an array of electrodes that is larger than the portion of the patient's skin that is being positioned against the array, according to the present disclosure.
Figure 11A illustrates locations on the left and right feet for SEM measurements according to the present disclosure.
Figure 11B is a plot of SEM values associated with known relative locations for identifying bisymmetric locations according to the present disclosure.
Figure 12A shows an exemplary configuration of a substrate shaped to be positioned in a known position on a patient's skin according to the present disclosure.
Figure 12B shows a front view of the exemplary configuration of Figure 12A according to the present disclosure.
Figure 13 depicts an integrated system for measurement, evaluation, storage, and transfer of SEM values, according to the present disclosure.

### DETAILED DESCRIPTION

This description is not intended to be a detailed catalog of all the different ways in which the disclosure may be implemented, or all the features that may be added to the instant disclosure. For example, features illustrated with respect to one embodiment may be incorporated into other embodiment, and features illustrated with respect to a particular embodiment may be deleted from that embodiment. Thus, the disclosure contemplates that in some embodiments of the disclosure, any feature or combination of features set forth herein can be excluded or omitted. In addition, numerous variations and additions to the various embodiments suggested herein will be apparent to those skilled in the art in light of the instant disclosure, which do not depart from the instant disclosure. In other instances, well-known structures, interfaces, and processes have not been shown in detail in order not to unnecessarily obscure the invention. It is intended that no part of this specification be construed to effect a disavowal of any part of the full scope of the invention. Hence, the following descriptions are intended to illustrate some particular embodiments of the disclosure, and not to exhaustively specify all permutations, combinations and variations thereof.

Unless otherwise defined, all technical and scientific terms used herein have the same meaning as commonly understood by one of ordinary skill in the art to which this disclosure belongs. The terminology used in the description of the disclosure herein is for the purpose of describing particular aspects or embodiments only and is not intended to be limiting of the disclosure.

All publications, patent applications, patents and other references cited herein are incorporated by reference in their entireties for the teachings relevant to the sentence and/or paragraph in which the reference is presented. References to techniques employed herein are intended to refer to the techniques as commonly understood in the art, including variations on those techniques or substitutions of equivalent techniques that would be apparent to one of skill in the art.

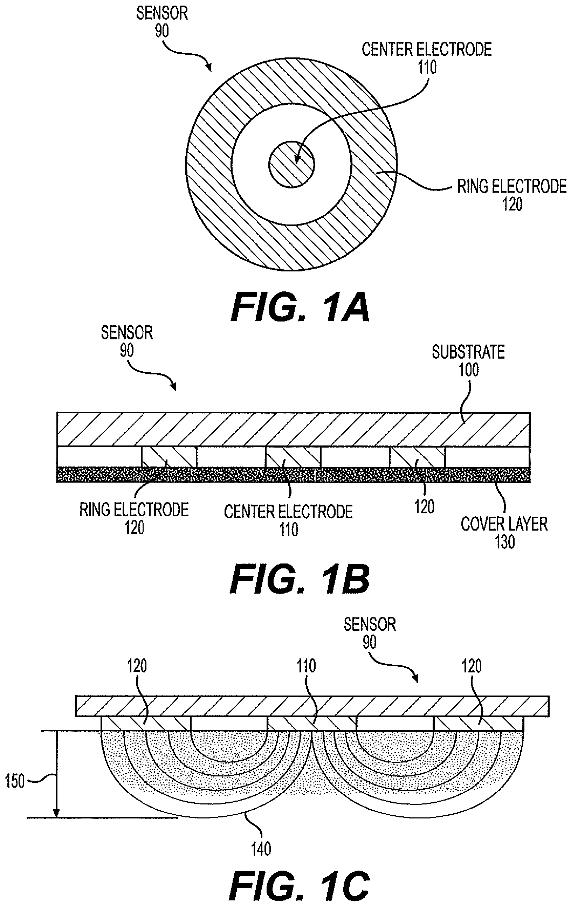

U.S. Patent Application Serial No. 14/827,375 discloses an apparatus that uses radio frequency (RF) energy to measure the sub-epidermal capacitance using a bipolar sensor similar to the sensor 90 shown in Figure 1, where the sub-epidermal capacitance corresponds to the moisture content of the target region of skin of a patient. The '375 application also discloses an array of these bipolar sensors of various sizes.

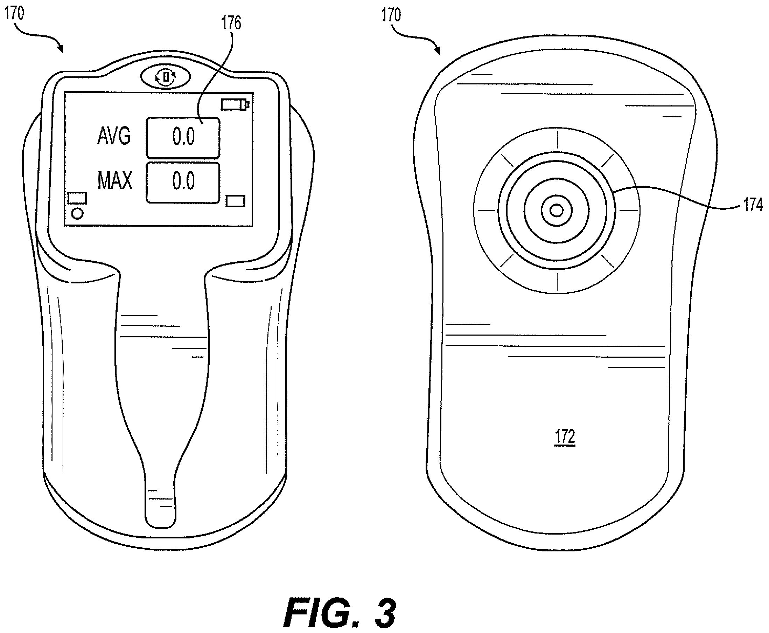

U.S. Patent Application Serial No. 15/134,110 discloses an apparatus for measuring sub-epidermal moisture (SEM) similar to the device shown in Figure 3, where the device emits and receives an RF signal at a frequency of 32 kHz through a single coaxial sensor and generates a bioimpedance signal, then converts this signal to a SEM value.

Both U.S. Patent Application Serial Nos. 14/827,375 and 15/134,110 are incorporated herein by reference in their entireties.

Unless the context indicates otherwise, it is specifically intended that the various features of the disclosure described herein can be used in any combination. Moreover, the present disclosure also contemplates that in some embodiments of the disclosure, any feature or combination of features set forth herein can be excluded or omitted.

The methods disclosed herein include and comprise one or more steps or actions for achieving the described method. The method steps and/or actions may be interchanged with one another without departing from the scope of the present invention. In other words, unless a specific order of steps or actions is required for proper operation of the embodiment, the order and/or use of specific steps and/or actions may be modified without departing from the scope of the present invention.

As used in the description of the disclosure and the appended claims, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

The terms "about" and "approximately" as used herein when referring to a measurable value such as a length, a frequency, or a SEM value and the like, is meant to encompass variations of ± 20%, ± 10%, ± 5%, ± 1%, ± 0.5%, or even ± 0.1% of the specified amount.

As used herein, phrases such as "between X and Y" and "between about X and Y" should be interpreted to include X and Y. As used herein, phrases such as "between about X and Y" mean "between about X and about Y" and phrases such as "from about X to Y" mean "from about X to about Y."

As used herein, the term "sub-epidermal moisture" or "SEM" refers to the increase in tissue fluid and local edema caused by vascular leakiness and other changes that modify the underlying structure of the damaged tissue in the presence of continued pressure on tissue, apoptosis, necrosis, and the inflammatory process.

As used herein, a "system" may be a collection of devices in wired or wireless communication with each other.

As used herein, "interrogate" refers to the use of radiofrequency energy to penetrate into a patient's skin.

As used herein, a "patient" may be a human or animal subject.

As used herein, "bisymmetric" refers to a pair of locations that are approximately equidistant from a line of symmetry.

As used herein, "delta" refers to a calculated difference between two SEM values.

Figure 1A is a plan view of a toroidal sensor 90 comprising a center electrode 110 and a ring electrode 120. In an aspect, electrodes 110 and 120 are disposed on a common surface of a substrate 100, as depicted in the cross-section of sensor 90 shown in Figure 1B. In one aspect, substrate 100 is rigid, for example a sheet of FR4 printed circuit board (PCB). In an aspect, substrate 100 is flexible, for example a sheet of polyimide. In one aspect, substrate 100 is a combination of rigid and flexible elements. In an aspect, electrodes 110 and 120 are covered with a cover layer 130 that is non-conductive so as to isolate electrodes 110 and 120 from each other and/or from external contact. In one aspect, portions of cover layer 130 are directionally conductive, enabling electrodes 110 and 120 to be in electrical contact with an object disposed on cover layer 130 while remaining electrically isolated from adjacent electrodes. In an aspect, cover layer 130 is rigid and planar, thereby providing a flat external surface. In one aspect, cover layer 130 conforms to the underlying electrodes 110 and 120 and substrate 100 such that there is no gap or air space between substrate 100 and cover layer 130. When an electric voltage is applied across electrodes 110 and 120, an electric field 140 is generated between electrodes 110 and 120 that extends outward from the plane of electrodes 110 and 120 to a distance 150, also referred to the depth of field, as shown in Figure 1C. The diameter of center electrode 110, the inner and outer diameters of ring electrode 120, and the gap between electrodes 110 and 120 may be varied to change characteristics of field 140, for example the depth of field 150.

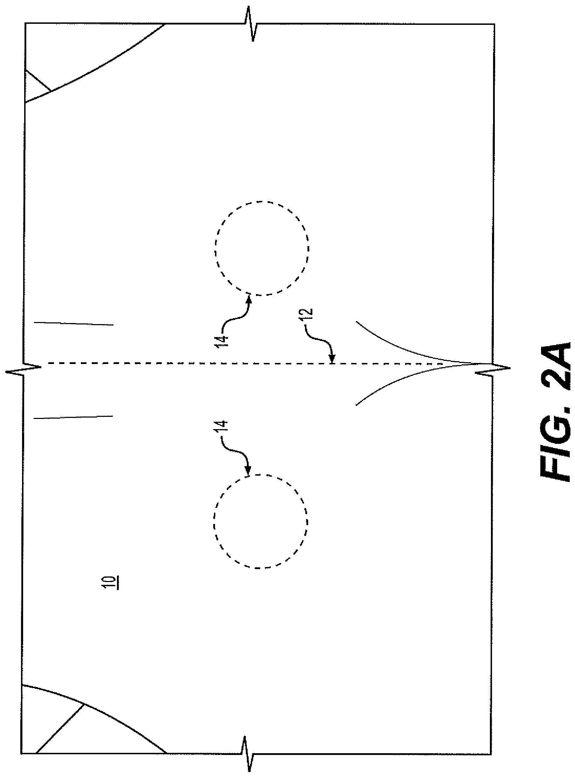

Figure 2A depicts the sacral region of the back of a patient 10. A line of symmetry 12 can be drawn down the center of the back, dividing the back into left and right mirror images. Locations 14 are approximately the same distance from line of symmetry 12 and approximately at the same height and are, therefore, considered to be bisymmetric locations on the back of patient 10.

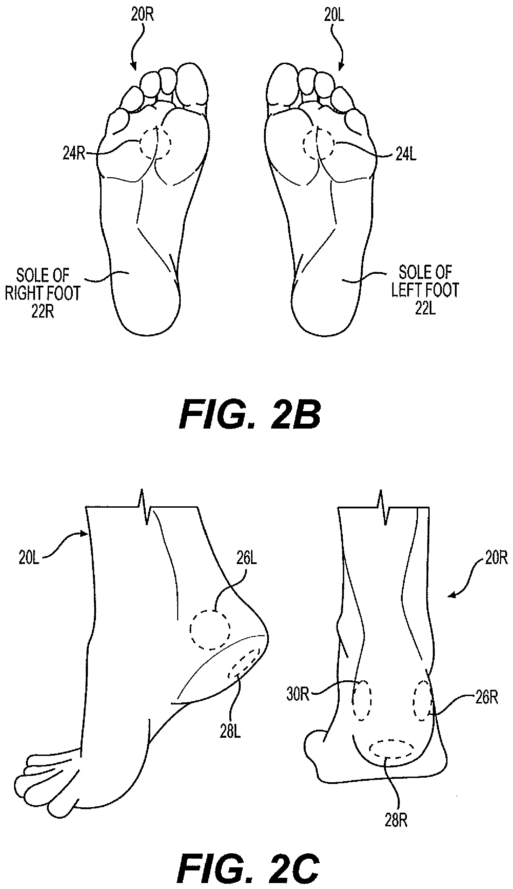

Figure 2B depicts left foot 20L and right foot 20R of a patient 10, as seen if patient 10 were lying on the back on a bed (not shown) and an observer were standing at the foot of the bed. With respect to soles 22L and 22R of feet 20L and 20R, locations 24L and 24R are located at approximately equivalent locations, e.g. the same distance from the posterior surface, i.e. the heel, and the same distance from the medial side of respective foot 20L or 20R and are considered to be bisymmetric locations.

Figure 2C depicts additional exemplary bisymmetric locations 26L and 26R located on the lateral sides of feet 20L and 20R, and bisymmetric locations 28L and 28R located on respective soles 22L and 22R of feet 20L and 20R. In an aspect, locations 26R and 30R are considered bisymmetric with respect to foot 20R when considered alone without reference to foot 20L.

Without being limited to a particular theory, comparison of SEM measurements taken at bisymmetric locations can compensate for an offset of readings of a particular patient from a population of patients. For example, a patient may be dehydrated on a particular day when measurements are being made. A comparison of the SEM value of healthy tissue from the same patient, while in a dehydrated condition, may be shifted from the SEM value of the same tissue at the same location when the patient is fully hydrated. If the tissue at one location is healthy while the tissue at the bisymmetric location is damaged, a comparison of the readings taken at the bisymmetric locations will exclude the "common mode" effect of dehydration on both locations and provide a more robust indication that tissue is damaged at one location.

Figure 3 depicts exemplary SEM measurement apparatus 170 comprising one toroidal sensor 174 disposed on underside 172 of an apparatus body. Apparatus 170 may be used to take measurements at multiple locations, for example a first measurement at a first location and a second measurement at a second location that is bisymmetric relative to the first location. In an aspect, apparatus 170 comprises a processor that can be configured by instructions stored on a non-transitory computer-readable medium to determine a characteristic of the measurements taken at multiple locations or parameters associated with or derived from the measurements, for example one or more of a difference between, an average of, or a difference of each from a common average of SEM values respectively derived from multiple measurements. In one aspect, apparatus 170 comprises a display configured to show one or more parameters associated with the measurements, for example a delta between SEM values derived from measurements taken at two bisymmetric locations.

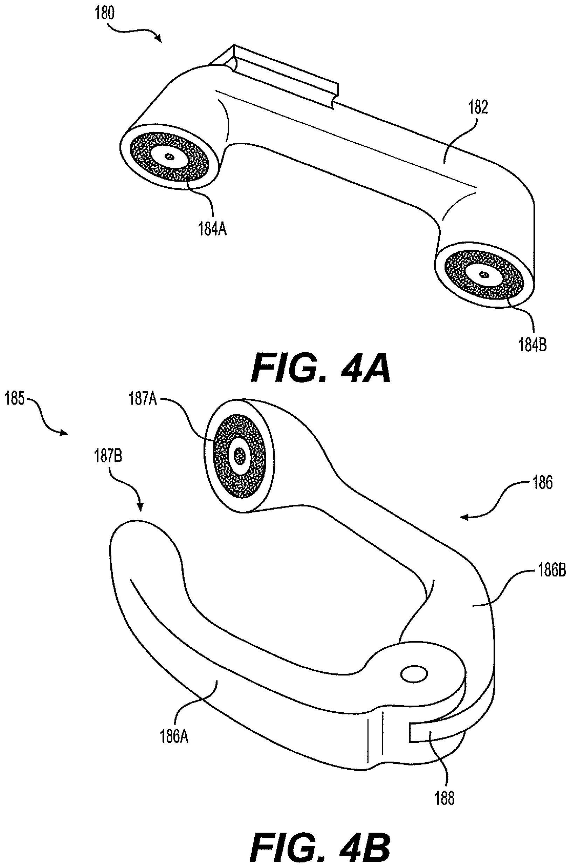

Figure 4A depicts an exemplary SEM measurement apparatus 180 comprising two sensors 184A and 184B located at separate locations on apparatus body 182, according to the present disclosure. An example usage would be to place apparatus 180 against a patient's body (not shown) so as to simultaneously position first sensor 184A at a first location and position second sensor 184B at a second location, both on the surface of a patient's skin. In an aspect, apparatus body 182 is rigid and maintains sensors 184A and 184B at a fixed separation distance and fixed orientation to each other. In one aspect, sensors 184A and 184B are aligned on a common plane, as shown in Figure 4A. In an aspect, apparatus body 182 is flexible such that sensors 184A and 184B may be oriented at an angle to each other. In one aspect, one or more of sensors 184A and 184B are movable such the angle between a movable sensor and the other sensor may be varied.

In use, apparatus 180 can measure an electrical property or parameter that comprises one or more electrical characteristics selected from the group consisting of a resistance, a capacitance, an inductance, an impedance, a reluctance, and other electrical characteristics with one or more sensors 184A and 184B. In an aspect, sensors 184A and 184B are configured as toroidal sensors such as shown in Figure 1A, with center electrode 110 and ring electrode 120. In one aspect, sensors 184A and 184B are provided in other configurations as discussed in this application. In an aspect, sensors 184A and 184B comprise an electrical ground plane (not shown) that is proximate to and separated from a portion of electrodes 110 and 120. In one aspect, a ground plane shields electrodes 110 and 120 from interference or modifies the shape of the field (similar in concept to field 140 of Figure 1C) of sensors 184A and 184B. In an aspect, a ground plane is disposed on a side of a substrate that is opposite the side on which electrodes 110 and 120 are disposed. In one aspect, apparatus 180 comprises a circuit (not shown) is electronically coupled to electrodes 110 and 120 of each sensor 184A and 184B and configured to measure an electrical property between electrodes 110 and 120. In an aspect, a ground plane is coupled to a ground or an equivalent floating reference of a circuit. In one aspect, a circuit is configured to determine and provide information regarding the measured electrical property. In an aspect, apparatus 180 takes the measurements with sensors 184A and 184B essentially simultaneously. In one aspect, apparatus 180 takes the measurements in sequence with a time interval between the measurements that ranges from zero to one second or more. In an aspect, a measurement by apparatus 180 is triggered by actuation of a button (not visible in Figure 4A) or an actuator. In one aspect, a measurement by apparatus 180 is triggered automatically based on input from a switching element (not shown in Figure 4A) that is part of apparatus 180, for example a contact sensor, a pressure sensor, an optical sensor, or other type of proximity-detecting device that is positioned, in an aspect, proximate to one or more of sensors 184A and 184B. In one aspect, multiple switching elements have to be simultaneously activated to provide the input to take the measurement.

In an aspect, apparatus 180 comprises a processor (not shown) that is coupled to a circuit and receives information about a measured electrical property from the circuit. In one aspect, information is in the form of an analog signal, e.g. an electrical voltage, or a digital signal. In an aspect, a processor is coupled directly to sensors 184A and 184B, and is configured to measure the electrical property directly. In one aspect, a processor is configured to convert the received electrical property into an SEM value. In an aspect, a processor is configured by machine-readable instructions that are stored on a non-transitory, computer-readable medium that is electronically coupled to the processor. In one aspect, instructions are loaded from a medium into a processor when apparatus 180 is powered on.

In an aspect, a measured electrical parameter is related to the moisture content of the epidermis of a patient at a depth that is determined by the geometry of the electrodes of sensors 184A and 184B, the frequency and strength of electrical field 140, with reference to Figure 1C, that is created by sensors 184A and 184B, and other operating characteristics of apparatus 180. In one aspect, the moisture content is equivalent to the SEM content with a value on a predetermined scale. In an aspect, a predetermined scale may range from 0 to 20, such as from 0 to 1, from 0 to 2, from 0 to 3, from 0 to 4, from 0 to 5, from 0 to 6, from 0 to 7, from 0 to 8, from 0 to 9, from 0 to 10, from 0 to 11, from 0 to 12, from 0 to 13, from 0 to 14, from 0 to 15, from 0 to 16, from 0 to 17, from 0 to 18, from 0 to 19. In one aspect, a predetermined scaled can be scaled by a factor or a multiple based on the values provided herein. In an aspect, multiple measurements are taken while varying one or more of operating characteristics between readings, thereby providing information related to the moisture content at various depths of the skin.

In an aspect, measurements of capacitance are taken simultaneously with sensors 184A and 184B when contact sensors (not visible in Figure 4A) determine that sensors 184A and 184B are in proper contact with two bisymmetric locations on a patient's skin. In an aspect, simultaneous capacitance measurements are compared to each other so as to determine whether the tissue under one of the bisymmetric locations is damaged. In one aspect, capacitance measurements are individually converted into SEM values that correspond to the moisture content of the tissue that is proximate to respective sensors 184A and 184B and the SEM values compared. In an aspect, a comparison is performed using equivalent voltages, capacitance values, or other intermediate signals.

In one aspect, a difference between SEM values is determined, where a difference that exceeds a predetermined threshold is indicative of tissue damage at one of the locations where the corresponding capacitance measurements were taken. In an aspect, means of SEM values obtained at each bisymmetric locations are determined and compared. In one aspect, medians or modes of SEM values obtained at each bisymmetric locations are determined and compared. In an aspect, the damage is indicated to be at the location associated with the larger of the SEM values. In one aspect, the damage is indicated to be at the location associated with the smaller of the SEM values. In an aspect, determination of whether there is tissue damage comprises one or more of comparison of individual SEM values with one or more predetermined ranges or thresholds and comparison of the difference with one or more predetermined ranges or thresholds. In an aspect, a predetermined range may be from 0.1 to 8.0, such as from 0.1 to 1.0, from 1.1 to 2.0, from 2.1 to 3.0, from 3.1 to 4.0, from 4.1 to 5.0, from 5.1 to 6.0, from 6.1 to 7.0, from 7.1 to 8.0, from 0.1 to 7.5, from 0.5 to 8.0, from 1.0 to 7.0, from 1.5 to 6.5, from 2.0 to 6.0, from 3.0 to 5.5, from 3.5 to 5.0, or from 4.0 to 4.5. In an aspect, a predetermined range may be from 0.1 to 4.0, such as from 0.5 to 4.0, from 0.1 to 3.5, from 1.0 to 3.5, from 1.5 to 4.0, from 1.5 to 3.5, from 2.0 to 4.0, from 2.5 to 3.5, from 2.0 to 3.0, from 2.0 to 2.5, or from 2.5 to 3.0. In one aspect, a predetermined range may be from 4.1 to 8.0, such as from 4.5 to 8.0, from 4.1 to 7.5, from 5.0 to 7.5, from 5.5 to 7.0, from 5.5 to 7.5, from 6.0 to 8.0, from 6.5 to 7.5, from 6.0 to 7.0, from 6.0 to 6.5, or from 6.5 to 7.0. In one aspect, a predetermined threshold may be about 0.3, 0.35, 0.4, 0.45, 0.5, 0.55, 0.6, 0.65, 0.7, 0.75, 0.8, 0.85, 0.9, 0.95, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, or 7.5. In one aspect, a predetermined threshold may range from 0.1 to 8.0, such as from 0.1 to 1.0, from 1.1 to 2.0, from 2.1 to 3.0, from 3.1 to 4.0, from 4.1 to 5.0, from 5.1 to 6.0, from 6.1 to 7.0, from 7.1 to 8.0, from 0.1 to 7.5, from 0.5 to 8.0, from 1.0 to 7.0, from 1.5 to 6.5, from 2.0 to 6.0, from 3.0 to 5.5, from 3.5 to 5.0, or from 4.0 to 4.5. In an aspect, a predetermined range or threshold can be scaled by a factor or a multiple based on the values provided herein. It will be understood that a predetermined value is not limited by design, but rather, one of ordinary skill in the art would be capable of choosing a predetermined value based on a given unit of SEM. In one aspect, ranges and thresholds of the present disclosure are varied according to the specific bisymmetric locations, the portion of a patient's body on which measurements are being made, or one or more characteristics of the patient such as age, height, weight, family history, ethnic group, and other physical characteristics or medical conditions.

One or more regions may be defined on a body. In an aspect, measurements made within a region are considered comparable to each other. A region may be defined as an area on the skin of the body wherein measurements may be taken at any point within the area. In an aspect, a region corresponds to an anatomical region (e.g., heel, ankle, lower back). In an aspect, a region may be defined as a set of two or more specific points relative to anatomical features wherein measurements are taken only at the specific points. In an aspect, a region may comprise a plurality of non-contiguous areas on the body. In an aspect, the set of specific locations may include points in multiple non-contiguous areas.

In an aspect, a region is defined by surface area. In an aspect, a region may be, for example, between 5 and 200 cm², between 5 and 100 cm², between 5 and 50 cm², or between 10 and 50 cm², between 10 and 25 cm², or between 5 and 25 cm².

In an aspect, measurements may be made in a specific pattern or portion thereof. In an aspect, the pattern of readings is made in a pattern with the target area of concern in the center. In an aspect, measurements are made in one or more circular patterns of increasing or decreasing size, T-shaped patterns, a set of specific locations, or randomly across a tissue or region. In an aspect, a pattern may be located on the body by defining a first measurement location of the pattern with respect to an anatomical feature with the remaining measurement locations of the pattern defined as offsets from the first measurement position.

In an aspect, a plurality of measurements are taken across a tissue or region and the difference between the lowest measurement value and the highest measurement value of the plurality of measurements is recorded as a delta value of that plurality of measurements. In an aspect, 3 or more, 4 or more, 5 or more, 6 or more, 7 or more, 8 or more, 9 or more, or 10 or more measurements are taken across a tissue or region.

In an aspect, a threshold may be established for at least one region. In an aspect, a threshold of 0.2, 0.3, 0.4, 0.5, 0.6, 0.7, 0.8, 0.9, or other value may be established for the at least one region. In an aspect, a delta value is identified as significant when the delta value of a plurality of measurements taken within a region meets or exceeds a threshold associated with that region. In an aspect, each of a plurality of regions has a different threshold. In an aspect, two or more regions may have a common threshold.

In an aspect, a threshold has both a delta value component and a chronological component, wherein a delta value is identified as significant when the delta value is greater than a predetermined numerical value for a predetermined portion of a time interval. In an aspect, the predetermined portion of a time interval is defined as a minimum of X days wherein a plurality of measurements taken that day produces a delta value greater than or equal to the predetermined numerical value within a total of Y contiguous days of measurement. In an aspect, the predetermined portion of a time interval may be defined as 1, 2, 3, 4, or 5 consecutive days on which a plurality of measurements taken that day produces a delta value that is greater than or equal to the predetermined numerical value. In an aspect, the predetermined portion of a time interval may be defined as some portion of a different specific time period (weeks, month, hours etc.).

In an aspect, a threshold has a trending aspect wherein changes in the delta values of consecutive pluralities of measurements are compared to each other. In an aspect, a trending threshold is defined as a predetermined change in delta value over a predetermined length of time, wherein a determination that the threshold has been met or exceeded is significant. In an aspect, a determination of significance will cause an alert to be issued. In an aspect, a trend line may be computed from a portion of the individual measurements of the consecutive pluralities of measurements. In an aspect, a trend line may be computed from a portion of the delta values of the consecutive pluralities of measurements.

In an aspect, the number of measurements taken within a single region may be less than the number of measurement locations defined in a pattern. In an aspect, a delta value will be calculated after a predetermined initial number of readings, which is less than the number of measurement locations defined in a pattern, have been taken in a region and after each additional reading in the same region, wherein additional readings are not taken once the delta value meets or exceeds the threshold associated with that region.

In an aspect, the number of measurements taken within a single region may exceed the number of measurement locations defined in a pattern. In an aspect, a delta value will be calculated after each additional reading.

In an aspect, a quality metric may be generated for each plurality of measurements. In an aspect, this quality metric is chosen to assess the repeatability of the measurements. In an aspect, this quality metric is chosen to assess the skill of the clinician that took the measurements. In an aspect, the quality metric may include one or more statistical parameters, for example an average, a mean, or a standard deviation. In an aspect, the quality metric may include one or more of a comparison of individual measurements to a predefined range. In an aspect, the quality metric may include comparison of the individual measurements to a pattern of values, for example comparison of the measurement values at predefined locations to ranges associated with each predefined location. In an aspect, the quality metric may include determination of which measurements are made over healthy tissue and one or more evaluations of consistency within this subset of "healthy" measurements, for example a range, a standard deviation, or other parameter.

In one aspect, a measurement, for example, a threshold value, is determined by SEM Scanner Model 200 (Bruin Biometrics, LLC, Los Angeles, CA). In another aspect, a measurement is determined by another SEM scanner.

In an aspect, a measurement value is based on a capacitance measurement by reference to a reference device. In an aspect, a capacitance measurement can depend on the location and other aspects of any electrode in a device. Such variations can be compared to a reference SEM device such as an SEM Scanner Model 200 (Bruin Biometrics, LLC, Los Angeles, CA). A person of ordinary skill in the art understands that the measurements set forth herein can be adjusted to accommodate a difference capacitance range by reference to a reference device.

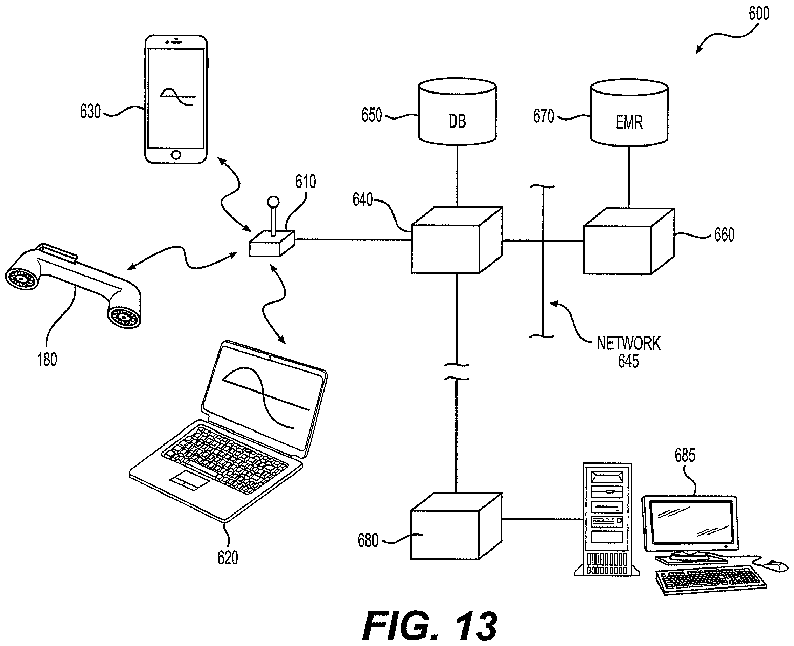

In an aspect, apparatus 180 is capable of storing multiple measurement and computation results. In one aspect, an apparatus in accordance with the present disclosure may also comprise other components, for example a camera or barcode scanner (not visible in Figure 4A), and may be capable of storing the output of that component. In an aspect, apparatus 180 comprises components (not visible in Figure 4A) to transfer the stored data, for example via a Bluetooth, WiFi, or Ethernet connection, to another device, for example a personal computer, server, tablet, or smart phone such as depicted in Figure 13.

Figure 4B depicts another aspect of an apparatus 186 that is configured to determine SEM values at bisymmetric locations. In an aspect, apparatus 186 comprises a hinge 188 such the separation distance between sensors 187A and 187B may be varied. In one aspect, sensors 184A and 184B are aligned with respect to apparatus body elements 186A and 186B to achieve a desired relative orientation, for example parallel to each other, at a predetermined separation distance. In an aspect, one or more of sensors 187A and 187B are movable such the angle between the movable sensor and the other sensor may vary, for example to match the orientation of the skin under each of sensors 187A and 187B as apparatus 185 is closed around an ankle to position sensors 187A and 187B over locations 26R and 30R shown in Figure 2C.

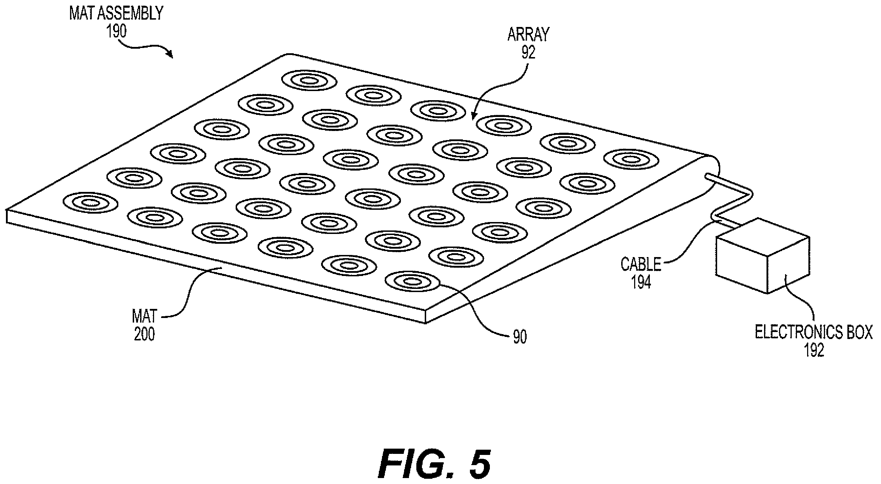

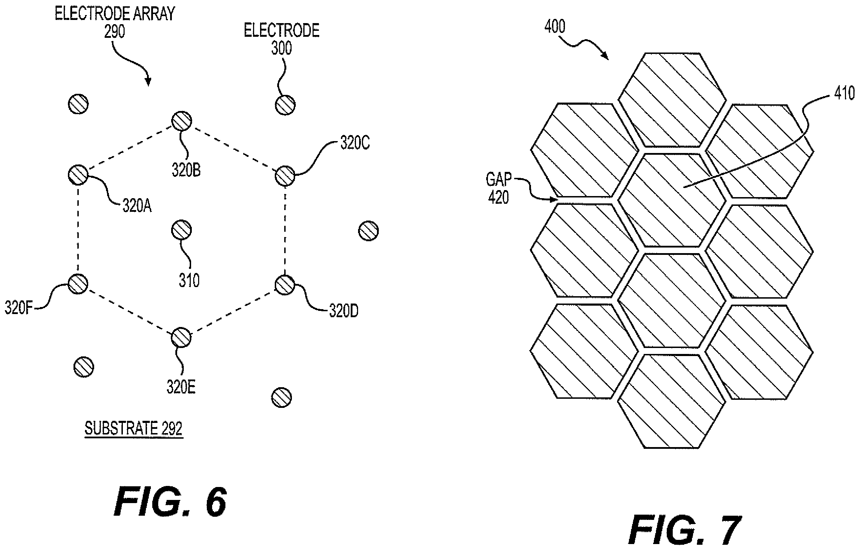

Figure 5 depicts an exemplary mat assembly 190 comprising array 92 comprising a plurality of sensors 90, according to the present disclosure. In one aspect, mat assembly 192 comprises a mat 200 on which sensors 90 are disposed. In an aspect, sensors 90 are embedded within mat 200. In one aspect, sensors 90 are located on the top surface of mat 200. In an aspect, sensors 90 have a cover layer (not visible in Figure 5) over them. In one aspect, sensors 90 comprise conductive electrodes that are exposed on their upper surface so as to create an electrical contact with an object proximate to the top of a mat, for example the feet of a patient standing on the mat. In an aspect, sensors 90 are toroidal sensors as shown in Figure 1A. In one aspect, sensors 90 are of a single type and configuration. In an aspect, sensors 90 vary in size and type within array 92. In one aspect, sensors 90 are of one or more alternate configurations, such as those discussed with respect to Figures 6, 7, 8A, and 8B. In an aspect, mat assembly 190 is coupled to an electronics assembly 192 either directly or through a cable 194. In one aspect, an electronics assembly 192 comprises a circuit (not visible in Figure 4A) coupled to electrodes of sensors 90 and a processor (not visible in Figure 4A) coupled to the circuit, as discussed previously with respect to apparatus 180.

In an aspect, mat assembly 190 comprises one or more of pressure sensors, temperature sensors, optical sensors, and contact sensors (not visible in Figure 5) disposed at one or more respective locations across mat 200. In one aspect, one or more measurements using sensors 90 are triggered by input from one or more of the pressure, temperature, optical, and contact sensors.

In an aspect, mat assembly 190 is configured as a floor mat and actuation of one or more of the pressure, temperature, optical, and contact sensors, for example detection of a person standing on mat assembly 190 due to detection of the weight of a person by a pressure sensor, initiates a measurement by one or more of sensors 90. In one aspect, sensors 90 are operated in a "detection mode" that is capable of detecting when a person steps onto mat assembly 190 and transitions into a "measurement mode" upon determination that a person is standing on mat assembly 190.

In an aspect, mat assembly 190 is configured as a portable apparatus that can be placed against a surface of a patient's skin, for example against a patient's back or against the soles of one or both of their feet while the patient is lying in bed. In one aspect, mat assembly 190 comprises one or more of a support tray, stiffening element, and conformal pad (not shown in Figure 5) to aid in placing sensors 90 against a surface of a patient's skin.

Figure 6 depicts an exemplary electrode array 290, according to the present disclosure. Array 290 is composed of individual electrodes 300 disposed, in this example, in a regular pattern over a substrate 292. In an aspect, each electrode 300 is separately coupled (through conductive elements not shown in Figures 6 through 8B) to a circuit, such as described with respect to Figure 4A, that is configured to measure an electrical parameter. In one aspect, a "virtual sensor" is created by selective connection of predetermined subsets of electrodes 300 to a common element of a circuit. In this example, a particular electrode 310 is connected as the center electrode, similar to electrode 110 of Figure 1A, and six electrodes 320A-320F are connected together as a "virtual ring" electrode, similar to electrode 120 of Figure 1A. In an aspect, two individual electrodes are individually connected to a circuit to form a virtual sensor, for example electrodes 310 and 320A are respectively connected as the two electrodes of a sensor. In one aspect, one or more electrodes 300 are connected together to form one or the other of the electrodes of a two-electrode sensor.

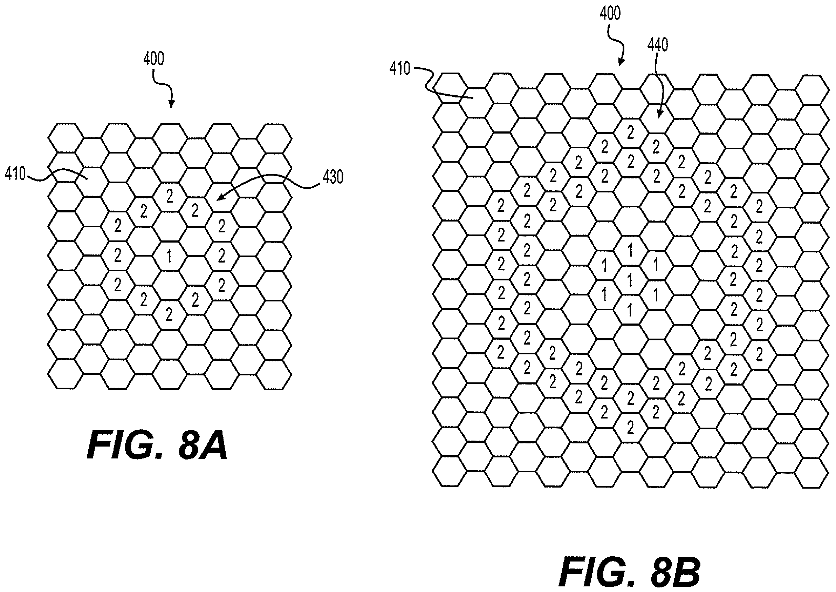

Figure 7 depicts another exemplary array 400 of electrodes 410, according to the present disclosure. In this example, each of electrodes 410 is an approximate hexagon that is separated from each of the surrounding electrodes 410 by a gap 420. In an aspect, electrodes 410 are one of circles, squares, pentagons, or other regular or irregular shapes. In one aspect, gap 420 is uniform between all electrodes 410. In an aspect, gap 420 varies between various electrodes. In one aspect, gap 420 has a width that is narrower than the cross-section of each of electrodes 410. In an aspect, electrodes 410 may be interconnected to form virtual sensors as described below with respect to Figures 8A and 8B.

Figure 8A depicts an array 400 of electrodes 410 that are configured, e.g. connected to a measurement circuit, to form an exemplary sensor 430, according to the present disclosure. In one aspect, a single hexagonal electrode 410 that is labeled with a "1" forms a center electrode and a ring of electrodes 410 that are marked with a "2" are interconnected to form a ring electrode. In an aspect, electrodes 410 between the center and ring electrode are electrically "floating." In one aspect, electrodes 410 between the center and ring electrode are grounded or connected to a floating ground. In an aspect, electrodes 410 that are outside the ring electrode are electrically "floating." In one aspect, electrodes 410 that are outside the virtual ring electrode are grounded or connected to a floating ground.

Figure 8B depicts an alternate aspect where array 400 of electrodes 410 has been configured to form a virtual sensor 440, according to the present disclosure. In an aspect, multiple electrodes 410, indicated by a "1," are interconnected to form a center electrode while a double-wide ring of electrodes, indicated by a "2," are interconnected to form a ring electrode. In one aspect, various numbers and positions of electrodes 410 are interconnected to form virtual electrodes of a variety of sizes and shapes.

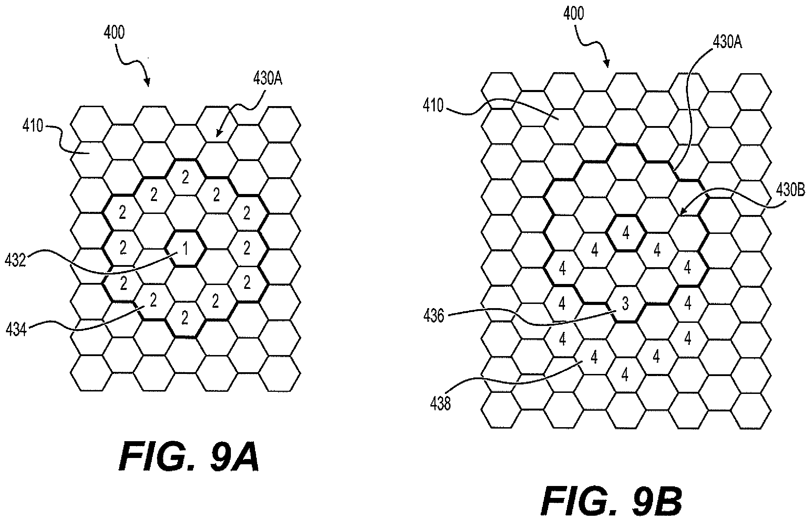

Figures 9A and 9B depict an exemplary configuration of an electrode array 400 that is capable of forming sensors 430 in multiple overlapping locations, according to the present disclosure. In Figure 9A, a virtual sensor 430A has been formed with center electrode 432 formed by a single electrode 410, indicated by a "1," and a ring electrode 434 formed by a plurality of electrodes 410, indicated by a "2." This same array 400 is shown in Figure 9B, where a new virtual sensor 430B has been formed with a center electrode 436, indicated by a "3," and ring electrode 438, indicated by a "4." The position of virtual sensor 430A is shown by a dark outline. It can be seen that virtual sensor 430B overlaps the position of virtual sensor 430A, allowing measurements to be made at a finer resolution than the diameter of sensors 430.

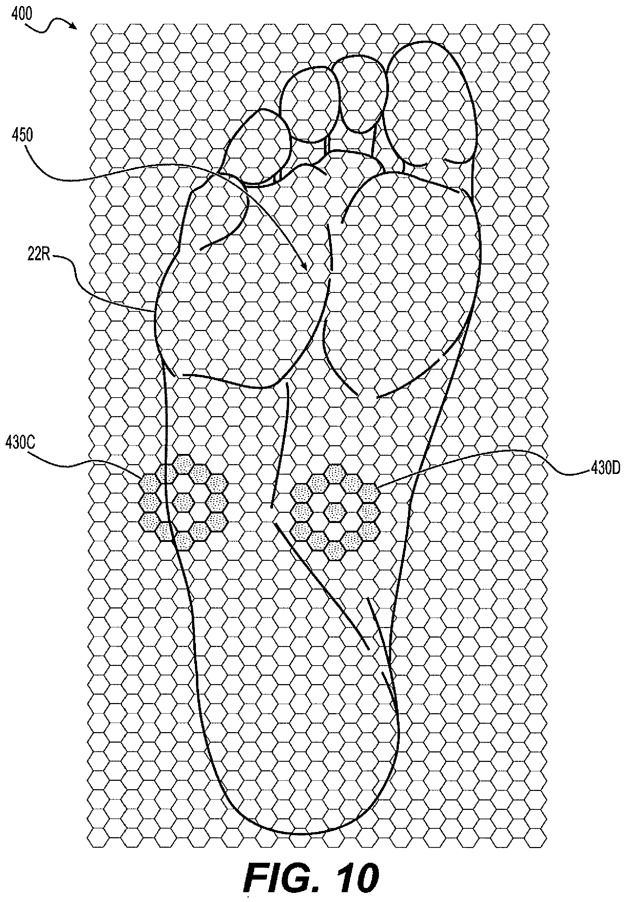

Figure 10 shows how sensors 430 may be formed from an array of electrodes 400 that is larger than the portion of a patient's skin that is being positioned against the array, according to the present disclosure. In this example, the outline of contact area 450 of sole 22R of right foot 20R of a patient 10, as seen from underneath foot 20R and with reference to Figures 2A-2C, is shown overlaid on array 400. In this example, sensor 430C has been formed in a location where a portion of sensor 430C extends beyond the edge of contact area 450. In such a position, capacitance or other electrical parameter measured by sensor 430C is lower than capacitance measured by sensor 430D, which is positioned completely within contact area 450. It can be seen that a sensor 430 may be formed at any point within array 400 and, depending on the position of sensor 430, may partially overlap the contact area at any level within a range of 0-100%.

In an aspect, two sensors may overlap 0-50%, such as 0-10%, 5-15%, 10-20%, 15-25%, 20-30%, 25-35%, 30-40%, 35%-45%, 40-50%, 0-25%, 15-35%, or 25-50%. In one aspect, two sensors may overlap 25-75%, such as 25-35%, 30-40%, 35%-45%, 40-50% 45-55%, 50-60%, 55-65%, 60-70%, 65-75%, 25-50%, 40-55%, or 50-75%. In one aspect, two sensors may overlap 50-100%, such as 50-60%, 55-65%, 60-70%, 65-75%, 70-80%, 75%-85%, 80-90%, 85-95%, 90-100%, 50-75%, 65-85%, or 75-100%.

In one aspect, an array of sensors 400 may further comprise a plurality of contact sensors (not shown on Figure 10) on the same planar surface as, and surrounding, each of the electrodes to ensure complete contact of the one or more virtual sensors to the skin surface. The plurality of contact sensors may be a plurality of pressure sensors, a plurality of light sensors, a plurality of temperature sensors, a plurality of pH sensors, a plurality of perspiration sensors, a plurality of ultrasonic sensors, a plurality of bone growth stimulator sensors, or a plurality of a combination of these sensors. In some embodiments, the plurality of contact sensors may comprise four, five, six, seven, eight, nine, or ten or more contact sensors surrounding each electrode.

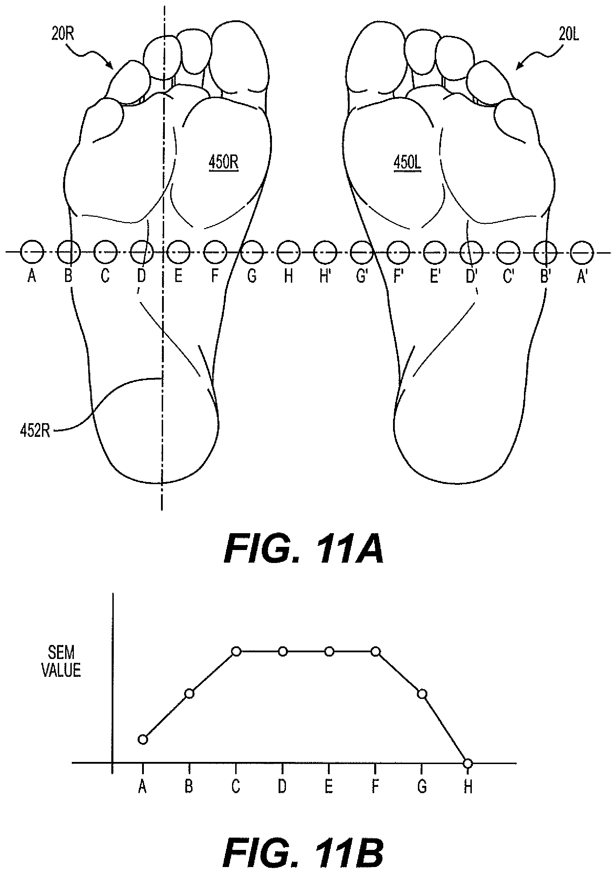

Figures 11A and 11B depict an example of how comparison of SEM values associated with sensors in known relative locations can identify bisymmetric locations, according to the present disclosure. In this example, sensors 430 are formed at nonoverlapping locations, marked "A" to "H" in Figure 11A, across a contact area 450R of a right foot 20R. The SEM values measured at each location are plotted in the graph of Figure 11B. In this example, the SEM value of locations "A" and "H" are low or zero, reflecting the non-overlap of sensor 430 with contact area 450 in those locations. The SEM values associated with locations "B" and "G" are higher, as sensor 430 overlaps a portion of contact area 450 in those positions. The SEM values for locations C-D-E-F are higher and, in this example, approximately the same, indicating that sensor 430 is completely within contact area 450 at those locations. In one aspect, an SEM measurement apparatus such as apparatus 180 may determine that certain locations, for example locations "C" and "F," are bisymmetric with respect to a centerline 452R of right foot 20R. In an aspect, where a similar set of measurements is made at locations A'-H' on left foot 20L, a location on each foot 20L and 20R, for example locations E and E', may be determined to be approximately bisymmetric.

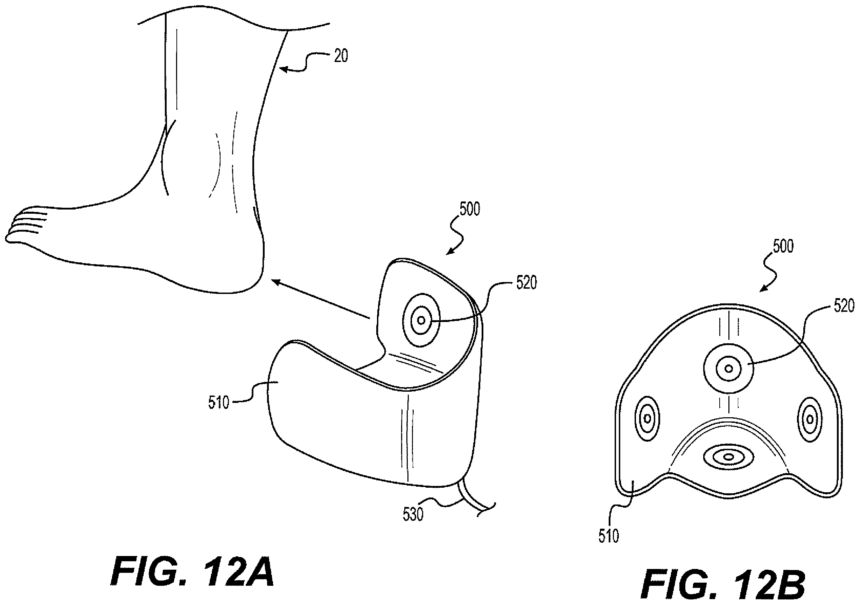

Figures 12A and 12B depict an exemplary aspect of a sensor assembly 500 configured to be placed in a known position on a patient's skin, according to the present disclosure. In this example, sensor assembly 500 has a shaped substrate 510 that is configured to conform to posterior and bottom surfaces of heel of a foot 20. In an aspect, shaped substrate 510 may be suitable for use with both a left foot 20L and a right foot 20R. In an aspect, sensor assembly 500 comprises one or more sensors 520 disposed on the inner surface of a shaped substrate 510. In this example, sensors 520 are configured as toroidal sensors as shown in Figure 1A. In one aspect, the inner surface of a shaped substrate 510 is lined with an array 400 of electrodes 410, with reference to Figure 7, such that virtual sensors may be formed at any location. In an aspect, sensors of other shapes and configurations are provided on the inner surface of a shaped substrate 510. In one aspect, shaped substrate 510 is a flexible panel (not shown in Figure 12A) that can be conformed to a patient's skin, for example wrapped around the back of an ankle. In an aspect, sensor assembly 500 comprises a cable 530 to connect sensors 520 to one or more of a power source, a circuit configured to measure one or more of capacitance or other electrical property, a processor, a communication subsystem, or other type of electronic assembly (not shown in Figure 12A).

Figure 12B depicts an exemplary configuration of sensor assembly 500 where multiple sensors 520 disposed on shaped substrate 510 such that, for example when sensor assembly 500 is placed against the skin of a patient around the back and bottom of the right heel, sensors 520 will be positioned in locations 26R, 28R, and 30R, with reference to Figure 2C, as well as on the center back of a heel. This enables multiple SEM measurements to be taken in repeatable location on a heel with sensor assembly 500 in a single position. In one aspect (not shown in Figures 12A and 12B), sensor assembly 500 is configured to be placed on a portion of the back of a patient thus providing the capability to make measurements at bisymmetric locations on the back. In an aspect, shaped substrate 510 is configured to match anatomical features of the target area of a patient. In an aspect, a shaped substrate 510 comprises markings or other indicators that can be aligned with features of a patient's body, so as to enable measurements to be taken at the same location at time intervals over a period of time in the general range of hours to weeks. In one aspect, sensor assembly 500 is integrated into a lining of a garment or shoe or other article of clothing. In one aspect, sensor assembly 500 is integrated into a sheet, blanket, liner, or other type of bed clothing. In an aspect, sensor assembly 500 comprises a wireless communication capability, for example a passive radio frequency identification (RFID) or an inductive coupling, to allow actuation of sensors 520 without physically connecting to sensor assembly 500.

Figure 13 depicts a schematic depiction of an integrated system 600 for measurement, evaluation, storage, and transfer of SEM values, according to the present disclosure. In this example, system 600 comprises a SEM measurement apparatus 180, as discussed with respect to Figure 4A, that comprises the capability to wirelessly communicate with a WiFi access point 610. Apparatus 180 communicates with one or more of a SEM application running on a server 640, an application running on a laptop computer 620, a smart phone 630, or other digital device. In one aspect, laptop computer 620 and smart phone 630 are carried by a user of apparatus 180, for example a nurse, and an application provides feedback and information to the user. In an aspect, information received from apparatus 180 for a patient is stored in a database 650. In one aspect, information received from apparatus 180 is transferred over a network 645 to another server 660 that stores a portion of information in an electronic medical record (EMR) 670 of a patient. In one aspect, information from apparatus 180 or retrieved from database 650 or EMR 670 is transferred to an external server 680 and then to a computer 685, for example a computer at the office of a doctor who is providing care for a patient.

From the foregoing, it will be appreciated that the present invention can be embodied in various ways, which include but are not limited to the following:

Embodiment 1. An apparatus for identifying damaged tissue, the apparatus comprising: a first sensor and a second sensor, where the first and second sensors each comprises a first electrode and a second electrode, and where each of the sensors is configured to be placed against a patient's skin, a circuit electronically coupled to the first and second electrodes and configured to measure an electrical property between the first and second electrodes of each of the sensors and provide information regarding the electrical property, a processor electronically coupled to the circuit and configured to receive the information from the circuit and convert the information into a sub-epidermal moisture (SEM) value, and a non-transitory computer-readable medium electronically coupled to the processor and comprising instructions stored thereon that, when executed on the processor, perform the step of: determining a difference between a first SEM value corresponding to the electrical property as measured by the first sensor at a first location on the patient's skin and a second SEM value corresponding to the electrical property as measured by the second sensor at a second location on the patient's skin, where the second location is bisymmetric relative to the first location.

Embodiment 2. The apparatus according to embodiment 1, where the difference being greater than a predetermined threshold is indicative of damaged tissue at one of the first and second locations.

Embodiment 3. The apparatus according to embodiment 1, where: the circuit is electronically coupled to the first and second electrodes of each of the first and second sensors, and the circuit is configured to convert a first electrical property measured with the first sensor into the first SEM value and convert a second electrical property measured with the second sensor into the second SEM value.

Embodiment 4. The apparatus according to embodiment 2, further comprising: a substrate configured to be placed in a known position on the patient's skin, and the first and second sensors are disposed on the substrate such that the first and second sensors are positioned at bisymmetric locations on the patient's skin when the substrate is placed in the known position on the patient's skin.

Embodiment 5. The apparatus according to embodiment 1, further comprising a gap between the first and second electrodes.

Embodiment 6. The apparatus according to embodiment 1, where the electrical property comprises one or more of an electrical component selected from the group consisting of a resistance, a capacitance, an inductance, an impedance, and a reluctance.

Embodiment 7. An apparatus for identifying damaged tissue, the apparatus comprising: a substrate configured to be placed against a surface of a patient's skin, a plurality of sensors that are disposed on the substrate at a respective plurality of positions, where each sensor comprises a pair of electrodes, a circuit electronically coupled to the pair of electrodes of each of the plurality of sensors and configured to measure an electrical property between the pairs of electrodes of a portion of the plurality of sensors and provide information regarding the measured electrical properties, a processor electronically coupled to the circuit and configured to receive the information regarding the electrical properties from the circuit and convert the plurality of electrical properties into a respective plurality of sub-epidermal moisture (SEM) values, and a non-transitory computer-readable medium electronically coupled to the processor and comprising instructions stored thereon that, when executed on the processor, perform the steps of: identifying from the plurality of SEM values a first sensor and a second sensor that are located at first and second positions that are bisymmetric with respect to the patient's skin, and comparing a first SEM value that is associated with the first sensor with a second SEM value that is associated with the second sensor.

Embodiment 8. The apparatus according to embodiment 7, where the instructions further comprise the steps of: determining a difference between the first and second SEM values, and providing an indication that tissue is damaged at one of the first and second locations if the difference is greater than a predetermined threshold.

Embodiment 9. The apparatus according to embodiment 7, where the instructions further comprise the steps of: determining a difference between the first and second SEM values, determining which of the first and second SEM values is larger than the other, and providing an indication that tissue is damaged at the location associated with the larger SEM value if the difference is greater than a predetermined threshold.

Embodiment 10. The apparatus according to embodiment 7, where the electrical property comprises one or more of an electrical component selected from the group consisting of a resistance, a capacitance, an inductance, an impedance, and a reluctance.

Embodiment 11. An apparatus for identifying damaged tissue, the apparatus comprising: an apparatus body; two sensors comprising a first sensor and a second sensor, where the two sensors are disposed on the apparatus body to allow simultaneous positioning of the first sensor on a first location on a patient's skin and the second sensor on a second location bisymmetric relative to the first location; a circuit electronically coupled to each of the two sensors and configured to measure an electrical property from each of the two sensors; a processor electronically coupled to the circuit and is configured to receive a first electrical property measurement from a first location and a second electrical property measurement from a second location, and to convert the first electrical property measurement to a first sub-epidermal moisture (SEM) value and the second electrical property measurement to a second SEM value; a non-transitory computer-readable medium electronically coupled to the processor and contains instructions that, when executed on the processor, perform the step of determining a difference between the first SEM value and the second SEM value.

Embodiment 12. The apparatus according to embodiment 11, where each of the two sensors are disposed on two ends of the apparatus body while being aligned on a common plane.

Embodiment 13. The apparatus according to embodiment 11, where the apparatus body is rigid and maintains the two sensors at a fixed separation distance and fixed orientation to each other.

Embodiment 14. The apparatus according to embodiment 11, where the apparatus body is flexible and allows the two sensors to be oriented at an angle to each other.

Embodiment 15. The apparatus according to embodiment 14, where the apparatus body comprises a hinge.

Embodiment 16. The apparatus according to embodiment 11, where each of the two sensors comprises a first electrode and a second electrode separated by a gap.

Embodiment 17. The apparatus according to embodiment 16, where the electrical property is measured between the first electrode and the second electrode.

Embodiment 18. The apparatus according to embodiment 11, where each of the two sensors comprises a plurality of electrodes separated by a gap.

Embodiment 19. The apparatus according to embodiment 18, where the plurality of electrodes are selectively activated to form a virtual ring electrode and a virtual central electrode.

Embodiment 20. The apparatus according to embodiment 11, where the electrical property comprises one or more of an electrical characteristic selected from the group consisting of a resistance, a capacitance, an inductance, an impedance, and a reluctance.

Embodiment 21. The apparatus according to embodiment 11, where the first electrical property measurement and the second electrical property measurement are measured simultaneously.

Embodiment 22. The apparatus according to embodiment 21, where the apparatus further comprises a contact sensor positioned proximate to one of the two sensors, and where the simultaneous measurements are triggered by the actuation of the contact sensor.

Embodiment 23. The apparatus according to embodiment 22, where the contact sensor is a pressure sensor or an optical sensor.

Embodiment 24. The apparatus according to embodiment 11, where the instructions further comprise the step of providing an indication that tissue is damaged at one of the first and second locations if the difference is greater than a predetermined threshold.

Embodiment 25. The apparatus according to embodiment 11, where the instructions further comprise the steps of: determining the greater of the first and second SEM values, and providing an indication that tissue is damaged at the location associated with the greater SEM value if the difference exceeds a predetermined threshold.

Embodiment 26. A method for identifying damaged tissue, the method comprising: obtaining a first sub-epidermal moisture (SEM) value from a first location on a patient's skin; obtaining a second SEM value from a second location that is bisymmetric relative to the first location; determining a difference between the first SEM value and the second SEM value.

Embodiment 27. The method according to embodiment 26, further comprising providing an indication that tissue is damaged at one of the first and second locations if the difference is greater than a predetermined threshold.

Embodiment 28. The method according to embodiment 26, further comprising: determining the greater of the first and second SEM values, and providing an indication that tissue is damaged at the location associated with the greater SEM value if the difference exceeds a predetermined threshold.

While the invention has been described with reference to particular aspects, it will be understood by those skilled in the art that various changes may be made and equivalents may be substituted for elements thereof without departing from the scope of the invention. In addition, many modifications may be made to a particular situation or material to the teachings of the invention without departing from the scope of the invention. Therefore, it is intended that the invention not be limited to the particular aspects disclosed but that the invention will include all aspects falling within the scope and spirit of the appended claims.

## Claims

1. An apparatus (500) for identifying damaged tissue, said apparatus comprising:
a shaped substrate (510) configured to conform to a patient's skin;
two or more sensors (520), each sensor comprising an array (400) of electrodes (410), wherein each electrode (410) is separated from each of the surrounding electrodes by a gap (420), and wherein said plurality of electrodes are selectively activated to form a first virtual ring electrode (434), first a virtual central electrode (432), a second virtual ring electrode, and a second virtual central electrode,
a circuit electronically coupled to said first and second virtual ring electrodes (434) and said first and second virtual central electrodes (432), configured to measure a first electrical property between said first virtual ring electrode and said first virtual central electrode, configured to measure a second electrical property between said second virtual ring electrode and said second virtual central electrode, and provide information regarding said first and second electrical properties;
a processor electronically coupled to said circuit and configured to receive said information; and
a non-transitory computer-readable medium electronically coupled to said processor and comprising instructions stored thereon that, when executed on said processor, perform the steps of:
converting said first electrical property into a first sub-epidermal moisture (SEM) value and said second electrical property into a second SEM value, and
determining a difference between said first SEM value and said second SEM value.

2. The apparatus according to claim 1, wherein said instructions further comprise a step of providing a signal if said difference is greater than a predetermined threshold.

3. The apparatus according to claim 1, further comprising a switching element configured to detect when said first and second sensors (520) are in proper contact with said patient's skin wherein:
said circuit is electronically coupled to said switching element and configured to measure said first and second electrical properties when said first and
second sensors (520) are in proper contact with said patient's skin.

4. The apparatus according to claim 1, wherein said gap (420) is uniform between all electrodes, is variable between various electrodes, or has a width that is narrower than the cross-section of each of the electrodes.

5. The apparatus according to claim 1, wherein said first and second electrical property comprises one or more electrical characteristics selected from the group consisting of a resistance, a capacitance, an inductance, an impedance, and a reluctance, wherein said first and second electrical property are the same.

6. The apparatus according to claim 1, wherein said apparatus further comprises a contact sensor positioned proximate to said two or more sensors (520), and wherein measurements of said first and second electrical properties are triggered at the same time by the actuation of said contact sensor.

7. The apparatus according to claim 6, wherein said contact sensor is a pressure sensor or an optical sensor.

8. The apparatus according to claim 1 for use in identifying damaged tissue, wherein said apparatus is configured to measure said first and second electrical properties simultaneously, and wherein the instructions further comprise the steps of:
obtaining said first SEM value from said first location on a patient's skin;
obtaining said second SEM value from said second location that is bisymmetric relative to said first location; and
determining said difference between said first and second SEM values.

9. The apparatus according to claim 8, wherein the instructions further comprise the step of providing an indication that tissue is damaged at one of said first and second locations if said difference is greater than a predetermined threshold.

10. The apparatus according to claim 8, wherein the instructions further comprise the steps of:
determining the greater of said first and second SEM values, and
providing an indication that tissue is damaged at the location associated with the greater SEM value if the difference exceeds a predetermined threshold.

11. The apparatus according to claim 1, wherein said apparatus takes the measurements of said first electrical property and said second electrical property essentially simultaneously.

12. The apparatus according to claim 5, wherein said first and second electrical properties comprise a capacitance.

13. The apparatus according to claim 1, wherein said apparatus further comprises a contact sensor positioned proximate to each of the said two or more sensors, wherein said first and second electrical properties are measured simultaneously, and wherein said simultaneous measurements are triggered by the actuation of both said contact sensors.
